# EUROPEAN PATENT SPECIFICATION

(11) **EP 2 400 878 B1**
(45) Date of publication and mention of the grant of the patent: **08.07.2020**
(21) Application number: 09803984.5
(22) Date of filing: 31.12.2009
(51) Int. Cl.: A61B 1/06, A61B 1/00

(54) **DISPOSABLE SHEATH FOR USE WITH AN IMAGING SYSTEM**
EINWEG-HÜLLE ZUR VERWENDUNG MIT EINEM BILDGEBUNGSSYSTEM
GAINE JETABLE POUR UTILISATION AVEC UN SYSTÈME D'IMAGERIE

(30) Priority: 24.02.2009 US 155003 P; 25.11.2009 US 625847
(43) Date of publication of application: 04.01.2012
(73) Proprietor: VisionScope Technologies LLC, Concord, MA 01742 (US)
(72) Inventor: CHEUNG, Lim, Concord MA 01742 (US); LESICA, Jeffrey, J., Arlington MA 02476 (US); PALOIAN, Michael, Cold Spring Harbor NY 11724 (US); ORCHARD, Anthony, Wantagh NY 11793 (US)
(74) Representative: Lock, Graham James
(86) International application number: PCT/US2009/069936
(87) International publication number: WO 2010/098807

(56) References cited:
- EP-A1- 0 647 425
- EP-A1- 0 672 379
- EP-A2- 0 280 397
- WO-A2-03/034905
- US-A- 5 704 892
- US-A1- 2002 013 513
- US-A1- 2002 087 047
- US-A1- 2006 069 314
- US-A1- 2008 064 925

## Description

### FIELD OF THE INVENTION

The present invention relates broadly to imaging systems and more specifically to disposable illumination sheaths for use with imaging systems.

### BACKGROUND OF THE INVENTION

This invention relates to imaging systems for use in minimally invasive surgical procedures. More particularly, this invention relates to a device for enhancing sterility in minimally invasive surgery. Even more particularly, this invention relates to a disposable illumination sheath having an illumination element. The disposable illumination sheath is usable with an endoscope that does not have an illumination element and in an associated surgical technique. An example of a disposable illumination pipe configured to be coupled with an endoscopic imaging system is described by document US 2002/013513.

Imaging devices such as endoscopes are for use in minimally invasive surgical procedures and are surgical instruments which enable a relatively non-intrusive visual inspection of internal body tissues. An endoscope includes a long tubular insertion member which is inserted into an opening in the body. The tubular insertion member of an endoscope generally includes optical fibers and lens systems for carrying organized visual information out of the patient, as well as a light source for illuminating the area to be visualized. Commercially available endoscopes designed for minimally invasive surgery employ a design in which the imaging elements are combined with the illumination elements in a single integrated entity. Such a combined endoscope cannot be used with an external disposable sterile barrier due to strong backscattered light that severely degrades image quality.

Because endoscopes are expensive instruments, they are used on multiple patients and must accordingly be sterilized after each procedure. Such sterilization procedures require substantial amounts of time, as well as expensive sterilization equipment and facilities. Costs are increased, not only because of the hospital personnel time involved, but also because the endoscopes are out of use for that additional time. Moreover, there is always the risk that sterilization is inadequate and that renegade bacteria or viruses remain in the endoscope and may be subsequently transferred to a patient.

Disposable systems to deal with these issues have been generally developed. Some systems employ disposable endoscopes whereby the entire endoscope consisting of both the imaging optics and illumination elements are discarded after each use. This approach, however, is expensive because high quality imaging optics are costly. Using lower grade, plastic imaging optics results in lower performance than traditional endoscopes. Other systems employ one-time use disposable sheaths that drape over an entire endoscope including both the imaging optics and the illumination light fibers. While such a system is less expensive, both the imaging optics and the light fibers are located behind the sheath's distal window. This results in backscatter of light off the window and into the imaging optics, degrading image quality to levels not competitive with traditional endoscopes.

Furthermore, some endoscopic techniques require extremely small points of entry so that a needle, for example, can be used to insert the endoscope. In order to maximize imaging capabilities within such a small space, a disposable sheath must be as thin as possible to enable the use of a larger imaging system within the endoscope.

Accordingly, there is a need for improved disposable endoscopic sheaths that are inexpensive and allow for high quality imaging.

### SUMMARY OF THE INVENTION

The present invention provides disposable devices for protecting imaging systems from contamination. More specifically, the present invention provides a disposable illumination sheath for use in an endoscopic imaging system having an optics housing coupled to a camera housing or handle with an imaging shaft extending distally from the optics housing, the optics housing comprising one or more proximal optics for transmitting image information viewed at a distal end of the imaging shaft to an imaging unit within the camera housing or handle; the disposable illumination sheath being for connection to the handle so as to cover the imaging shaft and the optics housing, and comprising:
an elongate tube for disposal over the imaging shaft having a sidewall with an inner surface, a proximal end, a distal end, a length, an inner diameter, and an outer diameter;
an illumination element provided along the length of the elongate tube; and
an optically transparent window sealed to the inner surface of the elongate tube proximate to the distal end;
wherein the illumination element consists of a solid tubular light transmitting structure, the illumination element being abuttable to an illumination source in the handle so as to transmit light to the distal end of the elongate tube for illuminating an imaging target, and
the elongate tube being sized so that the outer diameter is less than 3 mm and the inner diameter is greater than an outer diameter of the imaging shaft;
a barrier frame for disposal over the optics housing having a distal end coupled to the proximal end of the elongate tube, the barrier frame including a cavity formed therein sized to receive the optics housing of the endoscope imaging system; wherein a proximal end of the illumination element terminates in a port that is abuttable to the illumination source and positioned proximal to the solid tubular light transmitting structure.
In a preferred embodiment, the barrier frame comprises a sidewall containing a cylindrical light transmitting fiber that extends proximally from the solid tubular light transmitting structure.

In a preferred embodiment, the light transmitting tube includes a plurality of light transmitting fibers disposed therein.

In a preferred embodiment, light transmitted through the light transmitting tube is emitted through the distal end without traveling through the optically transparent window.

In a preferred embodiment, the elongate tube is opaque.

In a preferred embodiment, (a) the barrier frame includes a lumen formed through at least a portion thereof for receiving the imaging shaft; optionally wherein the lumen is axially aligned with the elongate tube; or (b) the barrier frame includes a proximal facing surface having a coupling mechanism disposed thereon for mating with the handle of the endoscopic imaging system; optionally wherein (i) the coupling mechanism comprises a bayonet mount or (ii) the illumination element extends through the barrier frame and includes a proximal connecting end that terminates on the proximal facing surface of the barrier frame; optionally wherein the coupling mechanism is configured to align the proximal connecting end of the illumination element with the illumination source in the handle when the barrier frame is coupled to the handle with a gap spacing between the proximal connecting end and the illumination source of less than 1 mm in the axial direction.

In a preferred embodiment, the outer diameter of the elongate tube is less than 2 mm and has an inner diameter of 1.5 mm.

In a preferred embodiment, the illumination element includes a proximal light connector that is abuttable to an illumination source through the handle so as to transmit light to the distal end of the elongate tube for illuminating an imaging target;
wherein the elongate tube is sized so that its outer diameter is less than about 2 mm; and
further comprising the barrier frame mated to a proximal portion of the elongate tube, the barrier frame having a proximal connecting element for removably connecting the sheath to the handle and aligning the light connector with an illumination source in the handle, wherein a gap spacing between the proximal light connector and the illumination source is less than 1 mm in the axial direction.

In a preferred embodiment, the illumination element comprises a plurality of optical fibers suspended within a substrate to form a solid light transmitting tube; optionally wherein (a) the plurality of optical fibers and the substrate are formed from a plastic material; or (b) the solid light transmitting tube has an outer diameter of less than 2 mm.

In a preferred embodiment, (a) the elongate tube is opaque; or (b) the elongate tube is formed from a metal.

In a preferred embodiment, the elongate tube has an inner diameter of 1.5 mm.

In a preferred embodiment, the proximal light connector of the illumination element is formed on a proximal facing surface of the barrier frame.

In a preferred embodiment, the proximal connecting element is a bayonet connector.

In a preferred embodiment, the disposable illumination sheath according to the invention further comprises a cylindrical structural element provided in an annular relationship with the solid tubular light transmitting structure; whereby when light is supplied to the proximal light connector, the light is transmitted through the solid tubular light transmitting structure and out its distal end; wherein the optically transparent window is sealed proximate to the distal end of the inner surface of the elongate tube; and wherein the light is transmitted through the distal end of the elongate tube without passing through the optically transparent window.

In a preferred embodiment, (a) the elongate tube has an outer diameter of less than 2 mm; or (b) the elongate tube has an inner diameter of 1.5 mm; or (c) the cylindrical structural element is opaque; or (d) wherein the barrier frame coupled to the proximal end of the elongate tube has a connector element for mating with an endoscopic camera housing; optionally wherein (a) the cylindrical illumination element extends through the barrier frame coupled to a sidewall thereof; or (b) the proximal light connector of the cylindrical illumination element is disposed on a proximal facing surface of the barrier frame.

In a preferred embodiment, the elongate tube comprises:
an opaque inner tubular member having a sidewall with an inner surface, a proximal end, a distal end, a length, an inner diameter, and an outer diameter, the elongate tube sized so that the inner diameter is 1.5 mm, the inner diameter being greater than an outer diameter of the imaging shaft; and
the solid tubular light transmitting structure is disposed concentrically around the inner tubular member and formed from a plurality of optical fibers suspended in a substrate in an elongate tubular shape, the solid tubular light transmitting structure sized to have an outer diameter that is less than 2 mm, the solid tubular light transmitting structure having a proximal connecting element for connecting to a light source in an endoscopic camera housing and a distal end for illuminating an imaging target; and wherein the barrier frame comprises:
   a. a hollow interior for receiving the endoscopic optics housing,
   b. a distal end coupled to the solid tubular light transmitting structure,
   c. a sidewall containing the cylindrical light transmitting fiber, and
   d. a mating connection formed on a proximal end and configured for mating with a corresponding connection on the endoscopic camera housing and aligning the proximal connecting element of the solid tubular light transmitting structure with a light source on the endoscopic camera housing with a gap spacing of less than 1 mm in the axial direction;
wherein light transmitted through the solid tubular light transmitting structure is transmitted through its distal end without traveling through the optically transparent window; optionally wherein (a) the barrier frame has a conical shape; or (b) the mating connection is a bayonet mating connection.

### BRIEF DESCRIPTION OF THE DRAWINGS

The invention will be more fully understood from the following detailed description taken in conjunction with the accompanying drawings, in which:
FIG. 1 is a perspective view of an exemplary disposable illumination sheath;
FIG. 2 is an exploded view of an exemplary disposable illumination sheath and imaging system;
FIG. 3 is another exploded view of an exemplary disposable illumination sheath and imaging system illustrating coupling mechanisms between the components;
FIG. 4 is a perspective view of an imaging system with an exemplary disposable illumination sheath disposed over the imaging system;
FIG. 5 is a perspective view of the imaging system of FIG. 4;
FIG. 6A is a perspective view of one embodiment of a disposable illumination sheath;
FIG. 6B is perspective view of an optics barrier of the disposable illumination sheath of FIG. 6A;
FIG. 6C is a cross-section view of one embodiment of an optics barrier of an exemplary disposable illumination sheath;
FIG. 7A is a front view of one embodiment of a coupling mechanism disposed on a camera housing of an imaging system for coupling with an exemplary disposable illumination sheath;
FIG. 7B is a cross-sectional view of the coupling mechanism of FIG. 7A;
FIG. 8A is a cross-sectional view of one embodiment of a distal end of an elongate tube of an exemplary disposable illumination sheath;
FIG. 8B is a cross-sectional view of the distal end of FIG. 8A;
FIG. 9 is a cross-sectional view of one embodiment of an optics barrier of an exemplary disposable illumination sheath;
FIG. 10A is a cross-sectional view of another embodiment of a distal end of an elongate tube of an exemplary disposable illumination sheath; and
FIG. 10B is a cross sectional view of the distal end of FIG. 10A.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments.

The present invention is generally directed to a disposable illumination sheath that provides a sterile barrier between an imaging device and a patient and which also illuminates an area under examination. The illumination sheath is designed specifically for use with an imaging system, for example, an endoscopic imaging system, that does not contain distal illumination elements, and can be designed to cover and/or enclose portions of the imaging device to prevent exposure to patient tissue and/or bodily fluids. An exemplary disposable illumination sheath of the present invention can be designed to cover and protect the imaging shaft, optics housing, camera housing, handle, imaging output and/or other electrical and component cords, as well as any other portion of an imaging device, such as an endoscopic device, that may be exposed to contamination. A used illumination sheath can be disposed of after a first procedure and a new, sterile illumination sheath can be used for a subsequent procedure. In this way, an imaging device can be utilized repeatedly without the need for a full sterilization between each procedure.

The disposable illumination sheath according to the invention includes an illumination element that transfers light from a light source within a handle or camera housing of an imaging device to a distal end of the disposable illumination sheath. The illumination element can generally be an elongate tube that forms a barrier between an imaging shaft of an imaging device and body tissue and fluids. The elongate tube of the illumination element can have a reduced wall thickness for maximizing available space within the disposable illumination sheath. In this way, a larger imaging system can be used within the sheath to increase the amount of image information collected by the imaging system. In some embodiments, the illumination element can be formed from a plastic material embedded with a plurality of plastic optical fibers having core and outer cladding materials that are compatible with the embedding material during a molding process. Such an illumination element can provide a particularly robust mechanism for transferring light at high efficiency while maintaining a thin profile to maximize interior space for imaging.

According to the invention, light from a light source within an imaging system can be transferred directly through an exemplary disposable illumination sheath to a distal end thereof. In many previous sheath systems, light from a source is transferred through an external pigtail that couples to the sheath in some way. The use of such a pigtail requires a connector into the sheath and can add weight and unwieldiness thereto. According to the present invention, light can be transferred from a light source within a handle and/or camera mount of an imaging system to the illumination element of a disposable illumination sheath. For example, the light source within the handle and/or camera mount can terminate in a flat, polished surface that is abuttable directly and/or with minimal gap spacing against a similarly flat, polished surface of the illumination element within the disposable illumination sheath. A proximal portion of the illumination element of the disposable illumination sheath can be embedded in a side wall of the barrier frame and can terminate on its proximal surface for mating with the light source. The polished surface of the light source and the polished surface of the illumination element can have matched areas and optical numerical apertures. In this way, the light transfer mechanism is permanently disposed internally within the sheath, thus obviating the need for an external pigtail and the problems associated therewith.

In further embodiments of the invention, an exemplary disposable illumination sheath can include a mating mechanism that can mate with an imaging system in such a way as to appropriately align the sheath with various aspects of the imaging system. For example, the mating mechanism can provide a coupling between the disposable illumination sheath and a camera housing of the imaging system that automatically aligns an illumination element within the sheath with a light source in the camera housing in the x-, y- and z- dimensions. The coupling can also ensure optical contact between the illumination element of the sheath and an exit aperture of the illumination source of the imaging system. The coupling can be, for example, a bayonet coupling that includes a rotational stop to indicate when the light source and the illumination element are properly aligned and the two components are properly coupled. In some embodiments, the bayonet connector on the camera housing can be integrally formed with a standard C-mount connector that enables coupling between the camera housing and the optics housing of the imaging system. This can prevent interference between the two couplings and can ensure optical contact between the illumination element of the disposable illumination sheath and the illumination source of the camera housing. This can further ensure proper spacing between an interior of the optics barrier and an exterior of the optics housing when both are coupled to the camera housing.

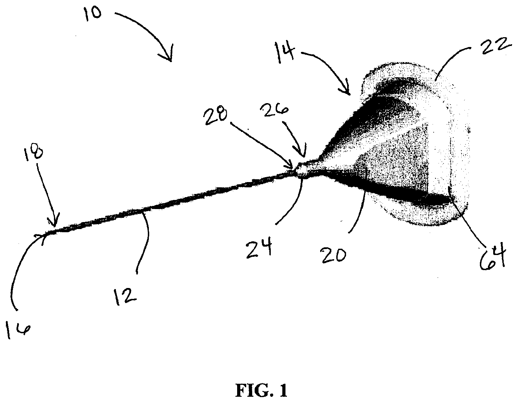

One embodiment of a disposable illumination sheath is illustrated in FIG. 1. A disposable illumination sheath 10 is provided that is generally configured to fit over an imaging device, such as an endoscope, that does not have an illumination element as an integral component. The sheath 10 can include an elongate tube 12 having a lumen therethrough that can receive an imaging shaft of the imaging device. The sheath 10 can also include a barrier portion 14 that can receive and enclose an imaging optics housing and/or camera housing. A distal window 16 can be disposed at a distal end 18 of the elongate tube 12. The distal window 16 can serve to seal the elongate tube 12 at its distal end 18 and can be optically transparent to allow imaging by the imaging device therethrough.

While the elongate tube 12 can have many configurations, in some embodiments, the elongate tube 12 can include a light transmitting element that is capable of transmitting light from a proximal light source to the distal end 18 of the sheath 10 to illuminate an area around the distal end 18 of the sheath 10. The light transmitting element can take a number of forms, some of which will be described in more detail below. In some embodiments, light that is transmitted through the light transmitting element can illuminate an area distal of the distal end 18 of the elongate tube 12 without passing through the distal window 16 in a proximal to distal direction. Such a configuration can prevent image degrading backscatter off of the distal window 16 and into the imaging device. For example, an opaque tube or other light blocking mechanism can be positioned between the light transmitting element and an inner lumen of the elongate tube 12 that receives the shaft of the imaging device. This can prevent light from entering the inner lumen of the elongate tube 12 and thus from passing in a proximal to distal direction through the distal window 16.

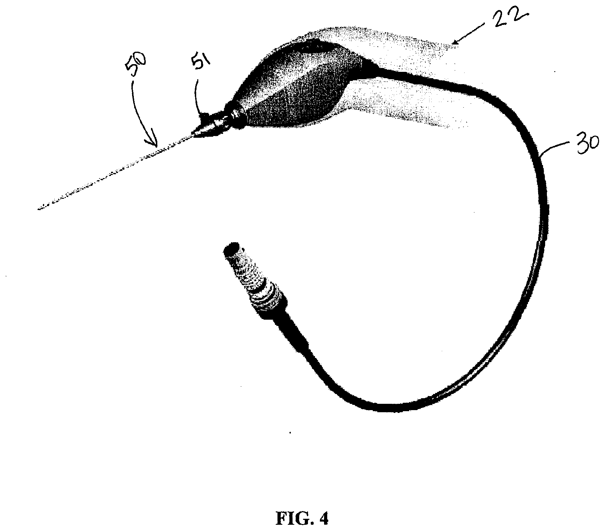

The barrier portion 14 of the sheath 10 can generally be formed of an optics barrier 20 (or barrier frame(20) and a camera barrier 22. The optics barrier 20 can receive and protect an imaging device optics housing, and the camera barrier 22 can receive and protect an imaging device camera housing. Both the optics barrier 20 and the camera barrier 22 can have any size and shape as needed to cover and/or enclose a particular imaging device optics housing and camera housing. In the embodiment illustrated in FIG. 1, a female connector 24 at a distal end 26 of the optics barrier 20 can couple to a proximal end 28 of the elongate tube 12. In the illustrated embodiment, the camera barrier 22 is a flexible material that is rolled into a compact form and coupled to a proximal portion 64 of the optics barrier 20. The camera barrier 22 can be deployed by unrolling it from its compact form to extend over and cover an imaging device camera housing. FIG. 4 shows one example of the camera barrier 22 in its deployed state covering an imaging device camera housing, as well as a portion of a connector such as an imaging output 30.

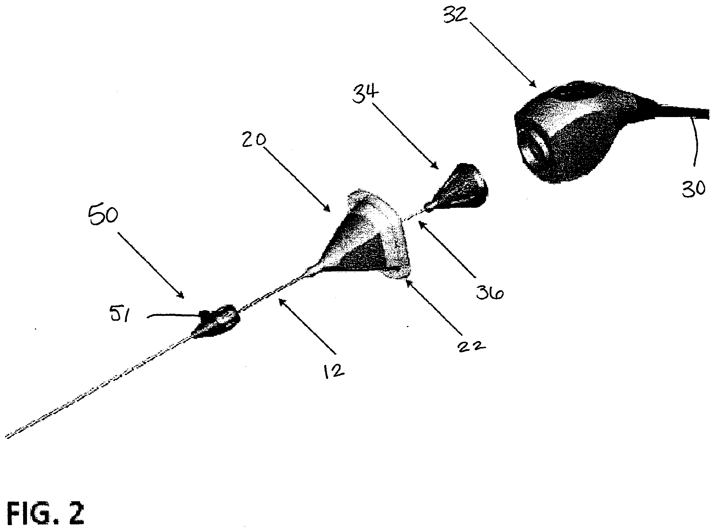

As noted above, the disposable illumination sheath 10 can be used with any imaging system known in the art including, but not limited to, endoscopic systems, ultrasonic systems, laparoscopic systems, etc. One exemplary imaging system in the form of an endoscopic imaging system is illustrated in FIGS. 2-5. The endoscopic system can generally include a camera housing 32 that can contain a high resolution imaging unit, such as a charge coupled device (CCD), a complementary metal-oxide-semiconductor (CMOS) imaging sensor, or any other pixilated flat panel sensor. The camera housing 32 can also include data processing electronics and any other electronics related to the imaging device. In addition, the camera housing 32 can house a power supply or a power input to provide power to the endoscope, as well as an illumination source for providing light to the light transmitting element of the disposable illumination sheath 10. The camera housing 32 can also act as a handle for controlling the endoscope and can include input controls for controlling the light source and for recording video and still images. A person skilled in the art will appreciate the variety of components and functions that the camera housing 32 can contain and perform.

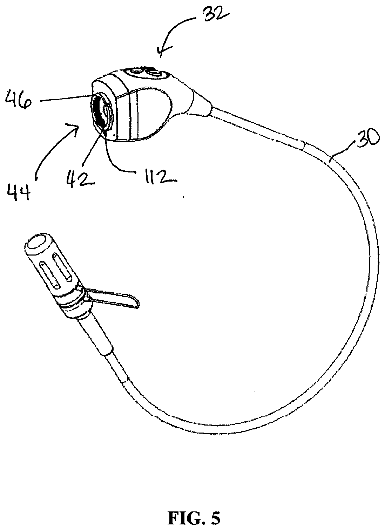

As shown most clearly in FIGS. 4 and 5, an imaging output 30 can extend from the camera housing 32 to an electronic storage and/or the display device, for example, a computer connected to a monitor. The imaging output 30 can transfer the electrical image from the imaging unit within the camera housing 32 to an electronic storage and/or the display device. The camera housing 32 can also be configured to transmit or receive wireless data to and/or from an external sensor, storage device, or display device.

Referring to FIG. 5, an illumination source disposed within the camera housing 32 is particularly advantageous in that no external inputs into the camera housing 32 or into the disposable illumination sheath 10 are required. Any illumination source known in the art can be utilized, including but not limited to, a light emitting diode of any power, a laser diode, a xenon light source, a halogen light source, etc. Furthermore, two or more different light sources can be included in the housing so that a user can selectively illuminate a region of interest with different wavelengths or bands of light. As will be described in more detail below, when the disposable illumination sheath 10 is coupled to the camera housing 32, a source port 112 for the illumination source can be automatically aligned with a light transmitting element in the disposable illumination sheath 10 so that light is transferred with minimal loss to the light transmitting element and through it to the distal end 18 of the sheath 10.

An exemplary endoscopic system can also include an optics housing 34 coupled to the camera housing 32 with an imaging shaft 36 extending distally therefrom. The imaging shaft 36 can contain one or more distal imaging optics positioned in a distal end thereof for viewing an interior of a patient's body. The optics housing 34 can contain one or more proximal optics for transmitting image information viewed at a distal end of the imaging shaft 36 to the imaging unit within the camera housing 32. A plurality of optical fibers can extend between the distal imaging optics in the imaging shaft 36 and the proximal optics within the optics housing 34 for transmitting the image information viewed by the distal imaging optics to the proximal optics. The optics housing 34 can receive the plurality of optical fibers and mate them to the proximal optics. Appropriate lens configurations for imaging within a body are well known in the art and any configuration can be used for both the distal imaging optics and the proximal optics as needed in a particular imaging system. The distal imaging optics should generally be configured for collecting image information at the distal end of the imaging shaft, while the proximal optics should generally be configured for projecting the image received at the proximal end of the plurality of optical fibers onto the imaging unit within the camera housing 32 for conversion into an electronic image.

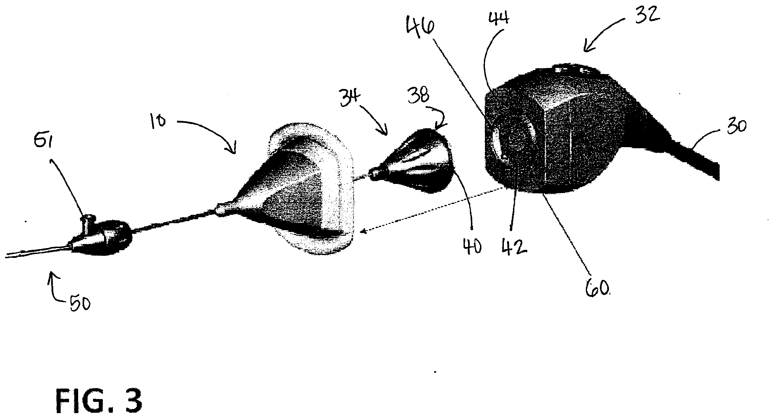

As seen most clearly in FIGS. 3, 5, and 7B, the optics housing 34 can mate to the camera housing 32 using a threaded connection, although any mating technique can be used. For example, a proximal surface 38 of the optics housing 34 can have a circular male threaded member 40 that can mate with a circular female threaded member 42 on a distal surface 44 of the camera housing 32. In the illustrated embodiment, the camera housing 32 includes a circular structure 46 having the threaded member 42 formed on an inside surface thereof. The male threaded member 40 on the optics housing 34 can be inserted into the circular structure 46 and threaded with the circular female threaded member 42 on the camera housing 32, sometimes referred to as a C-mount. A person skilled in the art will appreciate that any mating mechanism known in the art can be used to mate the optics housing and the camera housing, including an interference connection, a bayonet connection, a latch mechanism, etc.

As shown in FIGS. 2 and 3, the disposable illumination sheath 10 fits over the endoscopic system such that the elongate tube 12 of the disposable illumination sheath 10 encloses the imaging shaft 36 of the endoscope. The optics barrier 20 can enclose the optics housing 32, and the camera barrier 22 can be deployed to enclose the camera housing 32 and at least a portion of the imaging output 30.

As also shown in FIGS. 2 and 3, a traditional cannula 50 can be used to facilitate insertion of the disposable illumination sheath 10 and the endoscope into a body. As will be appreciated by those skilled in the art, in use, the cannula 50 can be inserted into a patient's body using standard techniques, for example, using an obturator or other puncture mechanism. Once inserted within the body, the obturator can be removed from the cannula 50, which then provides a working channel through tissue through which the disposable illumination sheath 10 and the endoscope can be inserted. In the most advantageous embodiments, the catheter is thin enough to be inserted as a needle. The cannula 50 can include features thereon, such as a luer lock 51, for injecting/removing drugs and fluids, and/or for insufflation. Once the endoscope or other imaging system is enclosed within and coupled to the disposable illumination sheath 10, the elongate tube 12 of the sheath 10 can be inserted into the cannula 50 to access a body cavity.

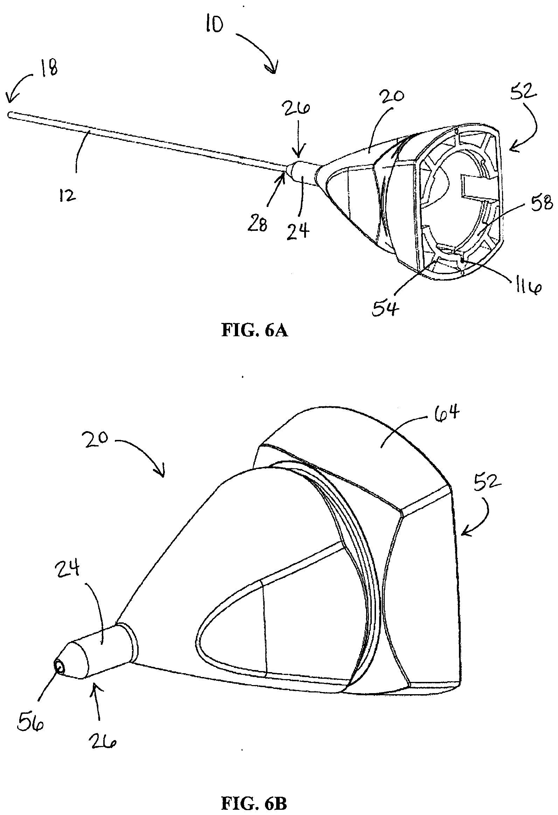

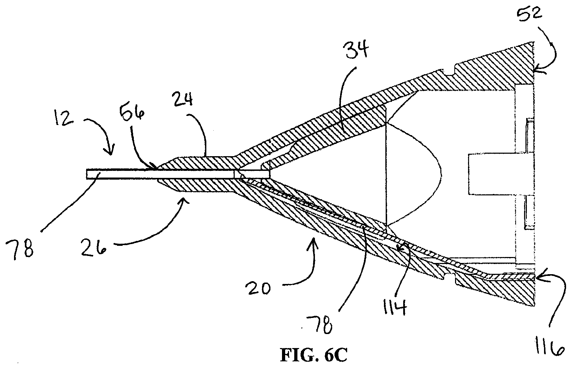

Referring now to FIGS. 6A-6C, the exemplary disposable illumination sheath 10 is illustrated in more detail. The optics barrier 20 can have any shape as needed to receive a particular optics housing, but in the illustrated embodiment, the optics barrier 20 has a generally conical shape. The conical shape of the barrier 20 can have one or more flattened sides, for example, two flattened sides, to better enable gripping of the device 10 by a user. A perimeter of the optics barrier 20 increases in size from the distal end 26 to a proximal surface 52. The optics barrier 20 can have an interior that is generally hollow for receiving the optics housing 34 therein. In some embodiments, a frame system 54 can be included within the interior of the optics barrier 20 for seating the optics housing 34 to provide stabilization thereto. As noted above, the distal end 26 of the optics barrier 20 can include a female connector 24 having an opening 56 formed therein for receiving the elongate tube 12. Any mating mechanism known in the art can be used to mate the opening 56 with the elongate tube 12, including, but not limited to, an adhesive, press fit, fasteners, etc. As shown most clearly in FIG. 6C, the opening 56 in the distal end 26 of the optics barrier 20 can form a lumen concentrically aligned with the lumen in the elongate tube 12 and extending into the interior of the optics barrier 20. As shown, the imaging shaft 36 can be inserted through the interior of the optics barrier 20 and through the opening 56 to extend into the elongate tube 12.

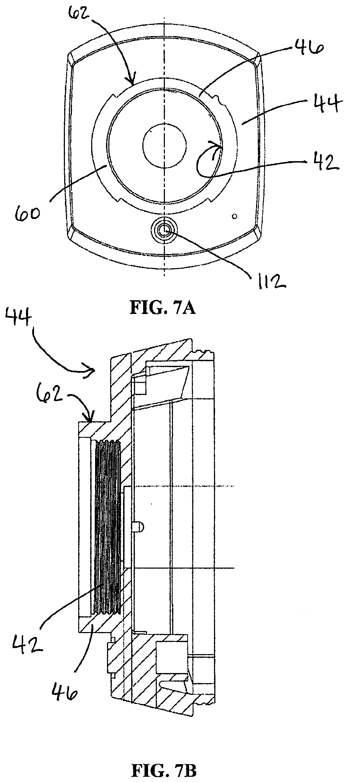

The proximal facing surface 52 of the optics barrier 20 can be configured to mate with the camera housing of an imaging system. Although any known mating mechanism can be used including a press fit, threads, interference fit, etc., in a preferred embodiment, a bayonet mating connection can be used. For example, the proximal facing surface 52 of the optics barrier 20 can include a female bayonet connector 58 for mating with a male bayonet connector 60 on the exemplary camera housing 32, illustrated in FIGS. 3 and 7A. In some embodiments, the male bayonet connector 60 can be integrally formed with the circular structure 46 on the camera housing 32. As noted above, the inner surface of the circular structure 46 can have the female threaded member 42 formed thereon for mating with the male threaded member 40 on the exemplary optics housing 34 (i.e., a C-mount). The male bayonet connector 60 can be formed, for example, on an outside surface 62 of the circular structure 46 such that both the female threaded member 42 and the male threaded member 40 are formed into single connector component. The bayonet mating connection can also have a positive stop and click registration feature so that the disposable illumination sheath 10 can be easily and consistently attached to the camera housing 32. This ensures that the coupling between the sheath 10 and the camera housing 32 is complete at a rotational position where the source port 112 is aligned with a fiber port 116 of the illumination element, as will be described in more detail below. In use, a user can first slide the sheath 10 over the imaging shaft 36 and the optics housing 34. The male and female bayonet connectors 58, 60 can be joined and a partial rotation of the sheath 10 relative to the camera housing 32 can complete the bayonet coupling such that all of the components are in proper alignment.

Referring back to FIGS. 1 and 4, the camera barrier 22 can take many forms, but should generally be suitable for shielding at least the camera housing 32. The camera barrier 22 can also be configured to shield at least a portion of the imaging output 30 and/or other cables extending from the camera housing 32. The camera barrier 22 can be a flexible material formed from any suitable material known in the art, including, but not limited to, polyurethane. As shown in FIG. 1, the camera barrier 22 can be rolled or otherwise secured in a compact form and coupled to the optics barrier 20 on a proximal portion 64 thereof for deployment after the disposable illumination sheath 10 has been positioned over the imaging shaft 36 and over the optics housing 34. The camera barrier 22 can be coupled to the optics barrier using any mating mechanism known in the art for forming a leak-proof bond, preferably through the use of radio-frequency welding. The camera barrier 22 can be unrolled or otherwise deployed from its compact configuration by a user and draped over all sides of the camera housing 32 as shown in FIG. 4 to thereby shield the camera housing 32 from contamination from body tissue and/or bodily fluids. In general, a user can operate the endoscope controls on the camera housing 32 through the camera barrier 22.

The disposable illumination sheath 10 can also include an elongate tube for shielding the endoscope. The elongate tube 12 can have a proximal end 28 and a distal end 18 with an elongate length extending therebetween. A lumen can be formed through the elongate tube 12 that is capable of receiving an imaging shaft of an imaging system. The elongate tube 12 can have any length as needed for covering the imaging shaft 36. For example, the elongate tube 12 can generally have any length between about 1 cm and about 60 cm, and preferably can have a length that is in the range of 4 cm to 30 cm (e.g., 4 cm, 5 cm...9 cm, 10 cm...15 cm...30 cm) and matched to the length of the imaging shaft within so that the image formed is not vignetted by the exterior elongate tube 12. The proximal end 28 can couple to the distal end 26 of the optics barrier 20, as noted above. The distal end 18 can be configured to allow the imaging shaft 36 inserted within the elongate tube 12 to view and record images therethrough.

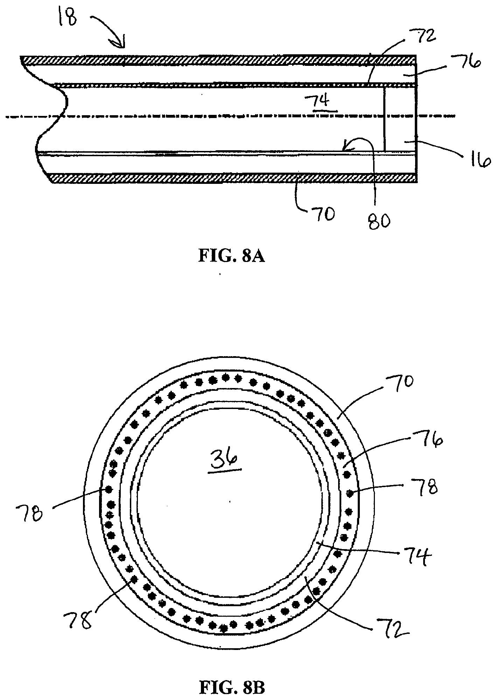

In some embodiments, the elongate tube 12 can be capable of transferring light from a light source to the distal end 18 of the elongate tube 12 to illuminate an area being imaged by the imaging system. Referring to FIG. 8, an enlarged view of the distal end 18 of an exemplary elongate tube 12 is illustrated. The elongate tube 12 can have an outer tubular member 70 which extends from within the optics barrier 20 and can serve as protective covering and a sterile barrier for the imaging shaft of the imaging device. An inner tubular member 72 can be spaced concentrically within the outer tubular member 70, and its inner lumen can define a cylindrical void or airspace 74 for receiving the imaging shaft 36 of the imaging system. The inner tubular member 72 can likewise extend from the distal end of the optics barrier 20. In a preferred embodiment, the outer tubular member 70 of the elongate tube 12 can be made from a stainless steel material, although any suitable material can be used.

The inner tubular member 72 can have a diameter less than a diameter of the outer tubular member 70 to create a channel 76 therebetween for receiving a plurality of illumination fibers 78. The illumination fibers 78 can be disposed concentrically about the inner tubular member 72 and can transmit light from an illumination source in the camera housing 32 and/or from an external illumination source through a fiber optic bundle that is connectable to the disposable illumination sheath 10. The illumination fibers 78 can be made from glass or plastic optical fibers, as will be appreciated by those skilled in the art. One or more layers of fibers 78 can be disposed within the channel 76 such that the channel 76 is generally filled with the fibers 78. The numerical aperture of the optical fibers 78 can be chosen to produce a light cone with an angle that matches or is slightly greater than the field of view of the distal imaging optics.

The illumination source, whether in the camera housing 32 or external thereto, can abut a proximal end of the illumination fibers 78 located between the outer tubular member 70 and the inner tubular member 72 of the elongate tube 12. For example, the illumination fibers 78 can extend out from between the inner and outer tubular members 72, 70 and into the optics barrier 20. The fibers 78 can be drawn together in a single circular bundle and can be embedded within and can extend along an inner surface 114 of a sidewall of the optics housing 20 or barrier frame 20, as shown in FIG. 6C. The bundle of fibers 78 can terminate on the proximal facing surface of the optics barrier 20 in a polished termination fiber port 116, preferably with a diameter of about 1 mm or less, although any diameter can be used. In one embodiment, an illumination source can be disposed within the camera housing 32 and can exit the camera housing 32 at the source port 112. As noted above, a bayonet mating connection between the optics barrier 20 and the camera housing 32 can ensure that the coupling between the sheath 10 and the camera housing 32 is complete at a rotational position where the source port 112 is aligned with a fiber port 116. In some embodiments, the fiber port 116 and the source port 112 are directly abuttable or abuttable with a gap spacing of less than 1 mm in the axial direction. Preferably, optical fibers leaving the camera housing 32 and optical fibers embedded within the optics barrier 20 can have identical numerical apertures so that optical entendre is conserved with a minimum amount of light loss across the coupling.
As noted above, the numerical aperture of the optical fibers can also be matched to the annular field of view of the distal imaging optics.

The distal window 16 can be sealed to an inner surface 80 of a distal-most end of the inner tubular member 72 to create a sterile barrier between the airspace 74 for receiving the imaging shaft 36 and the outer tubular member 70 which is in contact with the body. The distal window 16 can be flat and optically clear and as noted above, can be configured to allow the imaging system to view and record images therethrough. The distal window 16 can have any thickness as needed, but in one embodiment, can be about 0.5 mm thick. Of course, the distal window 16 can have a thickness less than 0.5 mm or greater than 0.5 mm, such as 1 mm, 2 mm, etc. Because the distal window 16 is sealed to the inner surface 80 of the opaque inner tubular member 72, light transmitted by the illumination fibers 78 does not travel through the distal window 16 in a proximal to distal direction, and rather only illuminates the area distal to the distal window 16. This prevents backscatter into the imaging shaft 36 that can degrade image quality. The disposable illumination sheath 10 can be configured with a stop or other mechanism near its proximal end so that the imaging shaft 36 of the imaging system is not over inserted into the disposable illumination sheath 10. For example, to prevent damage to the distal optics of the imaging shaft 36, the disposable illumination sheath 10 can be configured to allow for a longitudinal air gap between an interior surface of the distal window 16 and a distal surface of the imaging shaft 36 to accommodate dimensional tolerances associated with the manufacturing process. The thickness of the air gap is designed to fall within a minimum and a maximum allowable value, so that at the minimum, the inner distal optics will never come into contact with the interior surface of the distal window 16, and at the maximum, the distal imaging optics is not optically vignetted by the inner surface of the tube 72.

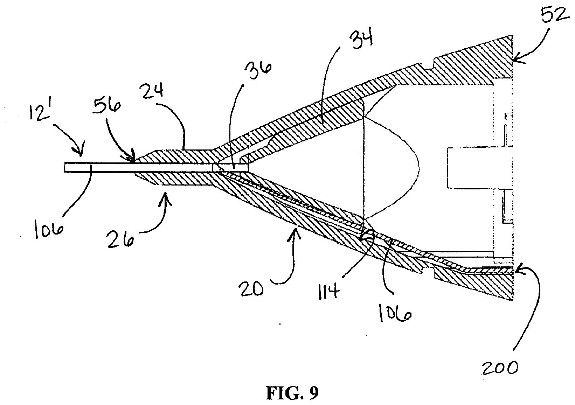

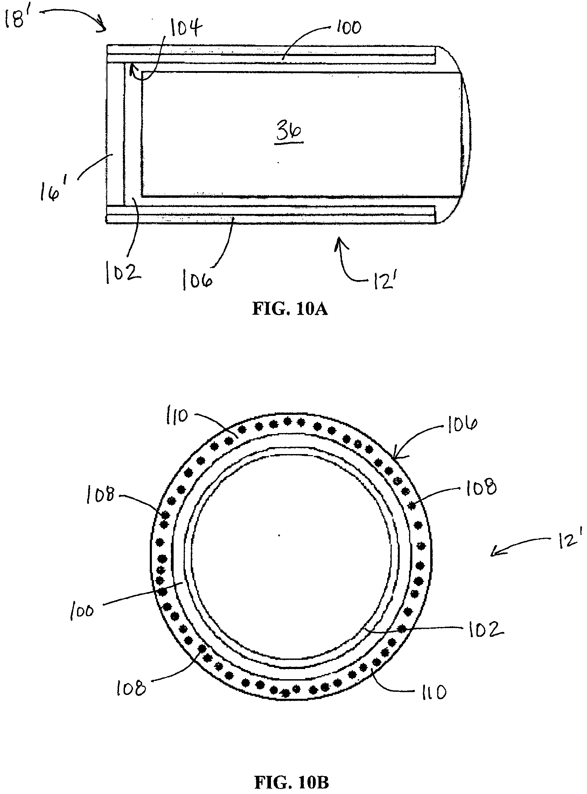

Referring now to FIGS. 9, 10A, and 10B, another exemplary light transmitting elongate tube 12' is provided. The elongate tube 12' can include an illumination element that is generally capable of transferring light to a distal end of the sheath 10. The illumination element can have many configurations and in some embodiments, the illumination element can be disposed around an opaque inner tubular member, as in the previous embodiment. In other embodiments, the illumination element is not disposed around an inner tubular member at all and can be utilized with or without an outer tubular member. The illumination element can be formed from many materials, and in one embodiment that will be described in more detail below, the illumination element is formed from a molded plastic material having a plurality of optical fibers embedded therein. The illumination element can also be formed of any light transmitting material formed into the shape of a tubular member and capable of transferring light from a source to the distal end of the sheath 10. The illumination element can preferably have a reduced wall thickness to provide the elongate tube 12' with an overall reduced thickness. The reduced wall thickness of the illumination element provides for more space within the elongate tube 12' so that a larger imaging system can be utilized while also reducing the overall diameter. A larger imaging system advantageously allows for the collection of more image information.

In the embodiment illustrated in FIGS. 9, 10A, and 10B, the illumination element is in the form of an illumination tube 106. Similar to the previous embodiment, the elongate tube 12' can include an opaque inner tubular member 100 that can create a cylindrical void or airspace 102 for receiving the imaging shaft 36 of the imaging system. A distal window 16' can be sealed to an inner surface 104 of a distal end of the inner tubular member 100 for sealing the airspace 102 from exposure to bodily tissue/fluids and for allowing imaging by the imaging system therethrough. The inner tubular member 100 can have a proximal end that couples to a distal end 26 of the optics barrier 20, as described in the previous embodiment.

The illumination tube 106 can be disposed concentrically around the inner tubular member 100 and can be formed from a plurality of plastic optical fibers 108 suspended and/or molded within a plastic material 110. The plurality of optical fibers 108 can have any diameter, but can generally have a diameter in the range of about 20 microns to 80 microns, and more preferably in the range of 30 microns to 50 microns. For example, as shown in FIG. 10B, a plurality of fibers 108 positioned in the shape of a ring can be drawn and molded within the plastic material 110 into a continuous tubular length. The plastic optical fibers 108 can be molded into any suitable material known in the art, including, but not limited to, a fluorinated polymer that is compatible with the cladding material of plastic fibers during the molding process. This can result in a solid tubular light transmitting structure that can be positioned around the inner tubular member 100 and can transfer light from an illumination source to a distal end 18' of the elongate tube 12'. The illumination tube 106 can have an inner surface in contact with an outer surface of the inner tubular member 100 and can be coupled to the outer surface of the inner tubular member 100 by, for example, compression fit or epoxy or other adhesive such that the inner tubular member 100 and the illumination tube 106 are an integrated structure.

The illumination tube 106 can have any size as need for a particular application, but in one embodiment, the illumination tube 106 can have an outer diameter of less than about 2 mm. It can have a thickness of less than about 0.5 mm such that the inner diameter of the illumination tube 106 is greater than about 1.5 mm. For example, in one embodiment, the outer diameter of the illumination tube 106 can be about 1.86 mm while the inner diameter of the illumination tube 106 is about 1.65 mm such that the thickness of the illumination tube 106 is about 0.21 mm. In other embodiments, the outer diameter of the illumination tube 106 can be in the range of 1.8 mm to 1.9 mm and the inner diameter can be in the range of 1.6 mm to 1.7 mm such that the thickness is in the range of 0.2 mm to 0.3 mm.

The inner tubular member 100 can have any size as need for a particular application, but in one embodiment, the inner tubular member 100 can have an outer diameter of less than about 2 mm and more preferably, of less than about 1.7 mm. It can have a thickness of less than about 0.5 mm, and more preferably, of less than about 0.2 mm, such that the inner diameter of the inner tubular member 100 is greater than about 1.4 mm and preferably greater than about 1.5 mm. For example, in one embodiment, the outer diameter of the inner tubular member 100 can be about 1.63 mm while the inner diameter of the inner tubular member 100 can be about 1.53 mm such that the thickness of the inner tubular member 100 is about 0.10 mm. In other embodiments, the outer diameter of the inner tubular member 100 can be in the range of 1.6 mm to 1.7 mm and the inner diameter can be in the range of 1.4 mm to 1.6 mm such that the thickness is in the range of 0.1 mm to 0.2 mm.

When combined into a unitary structure, the elongate tube 12' including the illumination tube 106 and the inner tubular member 100 can have any size as needed in a particular application. In one embodiment, the elongate tube 12' can have an outer diameter of less than about 2 mm and an inner diameter greater than about 1.4 mm for a thickness of about 0.6 mm. More particularly, the elongate tube 12' can have an outer diameter in the range of 1.8 mm to 1.9 mm, for example 1.86 mm. The inner diameter can be in the range of 1.4 mm to 1.6 mm, for example 1.63 mm. Therefore, the thickness of the elongate tube 12' can be in the range of 0.2 mm to 0.5 mm, for example, 0.23 mm.

Referring to FIG. 9, the elongate tube 12' including the illumination tube 106 and the inner tubular member 100 can extend into the female connector 24 on the optics barrier 20. While the inner tubular member 100 can terminate within the female connector 24, the illumination tube 106 can extend through the optics barrier 20 to couple with the source port 112 in the camera housing 32. For example, once the illumination tube 106 enters the female connector 24 of the optics barrier 20, it can split along one side and flatten so that it can mate to an inner surface 114 of the optics barrier 20 as shown in FIG. 9. The flattened illumination tube 106 can extend along the inner surface 114 of a sidewall of the optics barrier 20 and can terminate on the proximal surface 52 thereof. At a location distal to the proximal surface 52, the split sides of the illumination tube 106 can come together to form a tube again such that its mating surface terminates in a polished termination fiber port 200 as shown most clearly in FIG. 9.

As noted above, the proximal surface 52 of the optics barrier 20 can include a mating mechanism, such as a bayonet connector 58 for mating with the corresponding connector 60 on the distal surface 44 of the camera housing. The bayonet connector 58 can be configured to correctly align the fiber port 200 of the illumination tube 106 with the source port 112 in the camera housing 32 when the two are mated together such that a gap spacing between the two is less than 1 mm in the axial direction. Alignment of the fiber port 200 and the source port 112 allows for the illumination tube 106 to transmit light from the light source to illuminate an areas distal to the distal end of the disposable illumination sheath 12'. In addition, because of the opaque inner tubular member 100, light transmitted by the illumination tube 106 does not travel through the distal window 16' in a proximal to distal direction, thereby permitting backscattering into the imaging system and providing for a higher quality image.

There are many advantages to an illumination tube 106 such as that described herein. A illumination tube such as that described herein can be a more robust structure compared with glass fibers. Glass fibers can be fragile and can be difficult to assemble between two rigid tubular members. They can also be subject to breakage over time, reducing the quantity and quality of light delivered.

Furthermore, the illumination tube 106 in combination with the inner tubular member 100 results in an elongate tube 12' with a smaller profile when compared with the elongate tube 12 formed from glass fibers between two tubular members 70, 72. In particular, while the outer diameter of the two elongate tubes 12, 12' can be the same, the inner diameter of the elongate tube 12' can be larger than the inner diameter of the elongate tube 12 can be about. The maximum diameter of the imaging fiber bundle used in the imaging shaft 36 within the elongate tube 12 can be about 1 mm. The maximum diameter of the imaging fiber bundle used in the imaging shaft 36 within the elongate tube 12' can be about 1.2 mm. This allows the imaging fiber bundle used with the elongate tube 12' to be about 20% larger than that used with the elongate tube 12, allowing for the collection of about 20% more image information. This 20% increase in image information can be attained without requiring an increase in the outer diameter of the elongate tube 12'.

In use, a cannula or other access port can be inserted into a natural opening in the body or a surgical incision. A disposable illumination sheath 10 as disclosed herein can be placed over an endoscope or other imaging system such that the elongate tube 12, 12' is disposed over the imaging shaft 36 of the imaging system, and the optics barrier 20 is disposed over the optics housing 34. The bayonet mating connection between the optics barrier 20 and the camera housing 32 can be coupled such that all components are properly aligned. The camera barrier 22 can then be unrolled from its compact position such that it covers the camera housing 32 and a portion of the image output 30. The elongate tube 12, 12' can then be inserted into the cannula so that an area distal of the elongate tube 12, 12' can be illuminated using the illumination element on the elongate tube 12, 12' and imaged using the endoscope or other imaging system. Once the imaging is complete, the elongate tube 12, 12' can be withdrawn from the cannula. The disposable illumination sheath 10 can be uncoupled from the camera housing 32 and disposed of in an appropriate manner. If additional procedures are required, a new disposable illumination sheath 10 can be obtained and the endoscope or other imaging system can be reused.

A kit may be provided and can include any of the components described herein. For example, a kit can include an imaging system and a plurality of disposable illumination sheaths for use with the imaging system. A kit could also include any number of storage devices, display devices, power supplies, external light sources, if needed, and any number of electrical cords and image transfer cords. In other embodiments, a kit can contain only a plurality of disposable illumination sheaths configured to be used with a particular imaging system.

One skilled in the art will appreciate further features and advantages of the invention based on the above-described embodiments. Accordingly, the invention is not to be limited by what has been particularly shown and described, except as indicated by the appended claims.

## Claims

1. A disposable illumination sheath (10) for use in an endoscopic imaging system having an optics housing (34) coupled to a camera housing or handle (32) with an imaging shaft (36) extending distally from the optics housing, the optics housing comprising one or more proximal optics for transmitting image information viewed at a distal end of the imaging shaft (36) to an imaging unit within the camera housing or handle (32); the disposable illumination sheath being for connection to the handle so as to cover the imaging shaft (36) and the optics housing (34), and comprising:
an elongate tube (12; 12') for disposal over the imaging shaft (36) having a sidewall with an inner surface (104), a proximal end (28), a distal end (18; 26), a length, an inner diameter, and an outer diameter;
an illumination element (106) provided along the length of the elongate tube; and
an optically transparent window (16') sealed to the inner surface (104) of the elongate tube proximate to the distal end;
wherein the illumination element consists of a solid tubular light transmitting structure (106), the illumination element being abuttable to an illumination source in the handle so as to transmit light to the distal end of the elongate tube for illuminating an imaging target, and
the elongate tube being sized so that the outer diameter is less than 3 mm and the inner diameter is greater than an outer diameter of the imaging shaft (36);
a barrier frame (20) for disposal over the optics housing (34) having a distal end coupled to the proximal end of the elongate tube, the barrier frame including a cavity formed therein sized to receive the optics housing of the endoscope imaging system;
wherein a proximal end of the illumination element terminates in a port that is abuttable to the illumination source and positioned proximal to the solid tubular light transmitting structure.

2. The disposable illumination sheath of claim 1, wherein the barrier frame comprises a sidewall containing a cylindrical light transmitting fiber that extends proximally from the solid tubular light transmitting structure.

3. The disposable illumination sheath of claim 1, wherein the light transmitting tube includes a plurality of light transmitting fibers (108) disposed therein.

4. The disposable illumination sheath of claim 1, wherein light transmitted through the light transmitting tube is emitted through the distal end (18, 26) without traveling through the optically transparent window.

5. The disposable illumination sheath of claim 1, wherein the elongate tube (12; 12') is opaque.

6. The disposable illumination sheath of claim 1, wherein (a) the barrier frame includes a lumen formed through at least a portion thereof for receiving the imaging shaft (36); optionally wherein the lumen is axially aligned with the elongate tube (12; 12'); or (b) the barrier frame includes a proximal facing surface having a coupling mechanism disposed thereon for mating with the handle of the endoscopic imaging system; optionally wherein (i) the coupling mechanism comprises a bayonet mount (58) or (ii) the illumination element (106) extends through the barrier frame and includes a proximal connecting end that terminates on the proximal facing surface of the barrier frame; optionally wherein the coupling mechanism is configured to align the proximal connecting end of the illumination element (106) with the illumination source in the handle when the barrier frame is coupled to the handle (32) with a gap spacing between the proximal connecting end and the illumination source (112) of less than 1 mm in the axial direction.

7. The disposable illumination sheath of claim 1, wherein the outer diameter of the elongate tube (12; 12') is less than 2 mm and has an inner diameter of 1.5 mm.

8. The disposable illumination sheath of claim 1,
wherein the illumination element (106) includes a proximal light connector (200) that is abuttable to an illumination source through the handle so as to transmit light to the distal end of the elongate tube (12; 12') for illuminating an imaging target;
wherein the elongate tube (12; 12') is sized so that its outer diameter is less than about 2 mm; and
further comprising the barrier frame mated to a proximal portion of the elongate tube, the barrier frame having a proximal connecting element (58) for removably connecting the sheath to the handle and aligning the light connector with an illumination source (112) in the handle (32), wherein a gap spacing between the proximal light connector and the illumination source (112) is less than 1 mm in the axial direction.

9. The disposable illumination sheath of claim 8, wherein the illumination element (106) comprises a plurality of optical fibers suspended within a substrate to form a solid light transmitting tube; optionally wherein (a) the plurality of optical fibers and the substrate are formed from a plastic material; or (b) the solid light transmitting tube has an outer diameter of less than 2 mm.

10. The disposable illumination sheath of claim 8, wherein (a) the elongate tube (12; 12') is opaque; or (b) the elongate tube (12; 12') is formed from a metal.

11. The disposable illumination sheath of claim 8, wherein the elongate tube (12; 12') has an inner diameter of 1.5 mm.

12. The disposable illumination sheath of claim 8, wherein the proximal light connector of the illumination element is formed on a proximal facing surface of the barrier frame.

13. The disposable illumination sheath of claim 8, wherein the proximal connecting element (58) is a bayonet connector.

14. The disposable illumination sheath of claim 1, further comprising a cylindrical structural element (100) provided in an annular relationship with the solid tubular light transmitting structure (106);
whereby when light is supplied to the proximal light connector, the light is transmitted through the solid tubular light transmitting structure (106) and out its distal end; wherein the optically transparent window (16') is sealed proximate to the distal end (18; 26) of the inner surface of the elongate tube (12; 12'); and
wherein the light is transmitted through the distal end (18; 26) of the elongate tube (12; 12') without passing through the optically transparent window (16').

15. The disposable illumination sheath of claim 14, wherein (a) the elongate tube has an outer diameter of less than 2 mm; or (b) the elongate tube has an inner diameter of 1.5 mm; or (c) the cylindrical structural element is opaque; or
(d) wherein the barrier frame (20) coupled to the proximal end of the elongate tube has a connector element for mating with an endoscopic camera housing; optionally wherein (a) the cylindrical illumination element extends through the barrier frame (20) coupled to a sidewall thereof; or (b) the proximal light connector of the cylindrical illumination element is disposed on a proximal facing surface of the barrier frame.

16. The disposable illumination sheath of claim 1, wherein the elongate tube (12; 12') comprises:
an opaque inner tubular member (100) having a sidewall with an inner surface, a proximal end, a distal end, a length, an inner diameter, and an outer diameter, the elongate tube sized so that the inner diameter is 1.5 mm, the inner diameter being greater than an outer diameter of the imaging shaft (36); and
the solid tubular light transmitting structure (106) being disposed concentrically around the inner tubular member and formed from a plurality of optical fibers (108) suspended in a substrate (110) in an elongate tubular shape, the solid tubular light transmitting structure (106) sized to have an outer diameter that is less than 2 mm, the solid tubular light transmitting structure (106) having a proximal connecting element (200) for connecting to a light source (112) in an endoscopic camera housing (32) and a distal end for illuminating an imaging target; and wherein the barrier frame (20) comprises:
a. a hollow interior for receiving the endoscopic optics housing (34),
b. a distal end coupled to the solid tubular light transmitting structure (106),
c. a sidewall containing the cylindrical light transmitting fiber, and
d. a mating connection (58) formed on a proximal end and configured for mating with a corresponding connection (60) on the endoscopic camera housing (32) and aligning the proximal connecting element of the solid tubular light transmitting structure (106) with a light source on the endoscopic camera housing (32) with a gap spacing of less than 1 mm in the axial direction;
wherein light transmitted through the solid tubular light transmitting structure (106) is transmitted through its distal end without traveling through the optically transparent window; optionally wherein (a) the barrier frame (20) has a conical shape; or (b) the mating connection is a bayonet mating connection.

## Patentansprüche

1. Wegwerf-Beleuchtungshülle (10) zur Verwendung in einem endoskopischen Abbildungssystem, welches ein optisches Gehäuse (34) aufweist, das an ein Kamera-Gehäuse oder einen Griff (32) gekoppelt ist und eine Abbildungswelle (36) aufweist, die sich distal von dem optischen Gehäuse erstreckt, worin das optische Gehäuse ein oder mehrere proximale optische Elemente aufweist, um Bildinformationen, die an einem distalen Ende der Abbildungs-Welle (36) erfasst wurden, zu einer Bild-gebenden Einheit in dem Kamera-Gehäuse oder dem Griff (32) zu übertragen; worin die Wegwerf-Beleuchtungshülle für eine Verbindung mit dem Griff ausgestaltet ist, um die Abbildungs-Welle (36) und das optische Gehäuse (34) zu bedecken, und welche umfasst:
eine längliche Röhre (12; 12') zur Anordnung über die Abbildungs-Welle (36), welche aufweist, eine Seitenwand mit einer inneren Oberfläche (104), ein proximales Ende (28), ein distales Ende (18; 26), eine Länge, einen inneren Durchmesser, und einen äußeren Durchmesser;
ein Beleuchtungselement (106), das entlang der Länge der länglichen Röhre vorgesehen ist; und
ein optisch transparentes Fenster (16'), das an die innere Oberfläche (104) der länglichen Röhre nahe dem distale Ende abgedichtet vorgesehen ist;
worin das Beleuchtungselement aus einem festen röhrenförmigen Licht-Übertragungsaufbau (106) besteht, worin das Beleuchtungselement auf eine Beleuchtungsquelle in dem Griff auflegbar ist, um Licht zu dem distalen Ende der länglichen Röhre zu übertragen, um ein abzubildendes Ziel auszuleuchten, und
worin die längliche Röhre bemessen ist, so dass der äußere Durchmesser kleiner ist als 3 mm und der innere Durchmesser größer ist als ein äußerer Durchmesser der Abbildungs-Welle (36);
einen Absperrrahmen (20) zur Anordnung über dem optische Gehäuse (34), welches ein distales Ende aufweist, das mit dem proximalen Ende der länglichen Röhre gekoppelt ist, worin der Absperrrahmen eine Vertiefung beinhaltet, der darin bemessen ist, um das optische Gehäuse des Endoskop-Abbildungssystem aufzunehmen;
worin ein proximales Ende des Beleuchtungselements in einer Öffnung endet, die an der Beleuchtungsquelle angelegt und proximal zu dem festen röhrenförmigen Licht-Übertragungsaufbau angeordnet werden kann.

2. Wegwerf-Beleuchtungshülle nach Anspruch 1, worin der Absperrrahmen comprises eine Seitenwand mit einer zylindrischen Licht übertragenden Faser umfasst, die sich proximal von dem festen röhrenförmigen Licht-Übertragungsaufbau erstreckt.

3. Wegwerf-Beleuchtungshülle nach Anspruch 1, worin die Licht-übertragende Röhre mehrere darin vorgesehene Licht-übertragende Fasern (108) aufweist.

4. Wegwerf-Beleuchtungshülle nach Anspruch 1, worin das durch die Licht-übertragende Röhre übertragene Licht über das distale Ende (18, 26) ohne Durchgang durch das optisch transparente Fenster übertragen wird.

5. Wegwerf-Beleuchtungshülle nach Anspruch 1, worin die längliche Röhre (12; 12') lichtundurchlässig ist.

6. Wegwerf-Beleuchtungshülle nach Anspruch 1, worin (a) der Absperrrahmen ein Lumen beinhaltet, das durch mindestens einen Bereich davon ausgebildet ist, um die Abbildungs-Welle (36) aufzunehmen; wahlweise worin das Lumen axial mit der länglichen Röhre (12; 12') ausgerichtet ist; oder (b) worin der Absperrrahmen eine proximal ausgerichtete Oberfläche mit einem Kopplungsmechanismus aufweist, der darauf vorgesehen ist, um mit dem Griff des endoskopischen Abbildungssystems in Eingriff zu kommen; wahlweise worin (i) der Kopplungsmechanisums ein Bayonett-Verschluss (58) umfasst oder (ii) worin sich das Beleuchtungselement (106) durch den Absperrrahmen erstreckt und ein proximales Verbindungsende umfasst, das auf der proximal ausgerichteten Oberfläche des Absperrrahmens ausgerichtet ist; wahlweise worin der Kopplungsmechanismus ausgestaltet ist, das proximale Verbindungsende des Beleuchtungselement (106) mit der Beleuchtungsquelle in dem Griff zu verbinden, wenn der Absperrrahmen mit dem Griff (32) gekoppelt ist, worin ein Spaltabstand zwischen dem proximalen Verbindungsende und der Beleuchtungsquelle (112) kleiner als 1 mm in der axialen Richtung ist.

7. Wegwerf-Beleuchtungshülle nach Anspruch 1, worin der äußere Durchmesser der länglichen Röhre (12; 12') kleiner ist als 2 mm und einen inneren Durchmesser von 1,5 mm aufweist.

8. Wegwerf-Beleuchtungshülle nach Anspruch 1,
worin das Beleuchtungselement (106) einen proximalen Licht-Verbinder (200) aufweist, der an eine Beleuchtungsquelle durch den Griff anlegbar ist, um Licht zu dem distalen Ende der länglichen Röhre (12; 12') zu übertragen, um ein abzubildendes Ziel auszuleuchten;
worin die längliche Röhre (12; 12') bemessen ist, dass deren äußerer Durchmesser kleiner ist als etwa 2 mm; und
welche weiter den Absperrrahmen umfasst, der zu einem proximalen Bereich der länglichen Röhre passt, worin der Absperrrahmen ein proximales Verbindungselement (58) umfasst, um die Hüllen abnehmbar mit dem Griff zu verbinden und den Lichtverbinder mit einer Beleuchtungsquelle (112) in dem Griff (32) auszurichten, worin ein Spalt-Abstand zwischen dem proximalen Licht-Verbinder und der Beleuchtungsquelle (112) kleiner ist als 1 mm in der axialen Richtung.

9. Wegwerf-Beleuchtungshülle nach Anspruch 8, worin das Beleuchtungselement (106) mehrere optische Fasern umfasst, die in einem Substrat suspendiert sind, um eine feste, Licht-übertragende Röhre zu bilden; wahlweise worin (a) die mehreren optischen Fasern und das Substrat aus einem Kunststoffmaterial ausgebildet sind; oder (b) die feste Licht-übertragende Röhre einen äußeren Durchmesser von weniger als 2 mm aufweist.

10. Wegwerf-Beleuchtungshülle nach Anspruch 8, worin (a) die längliche Röhre (12; 12') lichtundurchlässig ist; oder (b) die längliche Röhre (12; 12') aus einem Metall gebildet ist.

11. Wegwerf-Beleuchtungshülle nach Anspruch 8, worin die längliche Röhre (12; 12') einen inneren Durchmesser von 1,5 mm aufweist.

12. Wegwerf-Beleuchtungshülle nach Anspruch 8, worin der proximale Licht-Verbinder des Beleuchtungselements auf einer proximal ausgerichteten Oberfläche des Absperrrahmens besteht.

13. Wegwerf-Beleuchtungshülle nach Anspruch 8, worin das proximale Verbindungselement (58) ein Bayonett-Verbinder ist.

14. Wegwerf-Beleuchtungshülle nach Anspruch 1, welche weiter ein zylindrisches Strukturelement (100) umfasst, das in einer ringförmigen Art und Weise mit dem festen röhrenförmigen Licht-Übertragungsaufbau (106) verbunden ist;
worin wenn Licht auf den proximalen Licht-Verbinder geworfen wird, das Licht über den festen röhrenförmigen Licht-Übertragungsaufbau (106) und aus dessen distalen Ende übertragen wird; worin das optische transparente Fenster (16') neben dem distalen Ende (18; 26) der inneren Oberfläche der länglichen Röhre (12; 12') abgedichtet vorgesehen ist; und
worin das Licht über das distale Ende (18; 26) der länglichen Röhre (12; 12') ohne durch das optisch transparente Fenster (16') zu gehen, übertragen wird.

15. Wegwerf-Beleuchtungshülle nach Anspruch 14, worin (a) die längliche Röhre einen äußeren Durchmesser von kleiner als 2 mm aufweist; oder (b) worin die längliche Röhre einen inneren Durchmesser von 1,5 mm aufweist; oder (c) worin das zylindrische Strukturelement lichtundurchlässig ist; oder (d) worin der mit dem proximalen Ende der längliche Röhre gekoppelte Absperrrahmen (20) ein Verbindungselement zur Anpassung an das an endoskopische Kameragehäuse aufweist; wahlweise worin (a) sich das zylindrische Beleuchtungselement durch den Absperrrahmen (20), gekoppelt an eine Seitenwand davon erstreckt; oder (b) der proximale Lichtverbinder des zylindrischen Beleuchtungselements auf einer proximal ausgerichteten Oberfläche des Absperrrahmens vorgesehen ist.

16. Wegwerf-Beleuchtungshülle nach Anspruch 1, worin die längliche Röhre (12; 12') umfasst:
ein lichtundurchlässiges inneres röhrenförmiges Element (100) mit einer Seitenwand mit einer inneren Oberfläche, einem proximalen Ende, einem distalen Ende, einer Länge, einem inneren Durchmesser, und einem äußeren Durchmesser, worin die längliche Röhre bemessen ist, so dass der innere Durchmesser 1,5 mm ist, der innere Durchmesser größer ist als ein äußerer Durchmesser der Abbildungs-Welle(36); und
worin der feste röhrenförmige Licht-Übertragungsaufbau (106) konzentrisch um das innere röhrenförmige Element angeordnet ist und aus mehreren optischen Fasern (108) besteht, die in einem Substrat (110) in einer länglichen röhrenförmigen Form suspendiert sind, worin der feste röhrenförmige Licht-Übertragungsaufbau (106) bemessen ist, einen äußeren Durchmesser aufzuweisen, der kleiner ist als 2 mm, worin der feste röhrenförmige Licht-Übertragungsaufbau (106) ein proximales Verbindungselement (200) aufweist, um mit einer Lichtquelle (112) in einem endoskopischen Kameragehäuse (32) zu verbinden und ein distales Ende zur Ausleuchtung eines abzubildenden Ziels; und worin der Absperrrahmen (20) umfasst:
a. einen hohlen Innenraum zur Aufnahme des endoskopischen optischen Gehäuses (34),
b. ein distales Ende, das mit dem festen röhrenförmigen Licht-Übertragungsaufbau (106) gekoppelt ist,
c. eine Seitenwand mit einer zylindrischen Licht-übertragenden Faser, und
d. eine Anpassverbindung (58), die an einem proximalen Ende ausgebildet und ausgestaltet ist, zu einer entsprechenden Verbindung (60) an dem endoskopischen Kameragehäuse (32) zu passen und Ausrichten des proximalen Verbindungselements des festen röhrenförmigen Licht-Übertragungsaufbaus (106) mit einer Lichtquelle an dem endoskopischen Kameragehäuse (32), wobei ein Spaltabstand kleiner ist als 1 mm in der axialen Richtung;
worin das durch den festen röhrenförmigen Licht-Übertragungsaufbau (106) übertragene Licht durch das distales Ende ohne Durchgang durch das optisch transparente Fenster übertragen wird; wahlweise worin (a) der Absperrrahmen (20) eine konische Form aufweist; oder (b) worin die Anpassverbindung ein Bayonettverschluss ist.

## Revendications

1. Gaine d'éclairage jetable (10) destinée à être utilisée dans un système d'imagerie endoscopique ayant un boîtier d'optique (34) couplé à un manche ou boîtier de caméra (32) avec une tige d'imagerie (36) s'étendant de manière distale à partir du boîtier d'optique, le boîtier d'optique comprenant une ou plusieurs optiques proximales pour transmettre des informations d'image, vues à une extrémité distale de la tige d'imagerie (36), à une unité d'imagerie à l'intérieur du manche ou boîtier de caméra (32) ; la gaine d'éclairage jetable étant destinée à être reliée au manche de façon à recouvrir la tige d'imagerie (36) et le boîtier d'optique (34), et comprenant :
un tube allongé (12 ; 12') destiné à être disposé par-dessus la tige d'imagerie (36), ayant une paroi latérale avec une surface interne (104), une extrémité proximale (28), une extrémité distale (18 ; 26), une longueur, un diamètre interne et un diamètre externe ;
un élément d'éclairage (106) disposé le long de la longueur du tube allongé ; et
une fenêtre optiquement transparente (16') scellée à la surface interne (104) du tube allongé à proximité de l'extrémité distale ;
l'élément d'éclairage consistant en une structure de transmission de lumière tubulaire pleine (106), l'élément d'éclairage étant apte à être en butée contre une source d'éclairage dans le manche de façon à transmettre de la lumière à l'extrémité distale du tube allongé pour éclairer une cible d'imagerie, et
le tube allongé étant dimensionné de telle sorte que le diamètre externe est de moins de 3 mm et que le diamètre interne est supérieur à un diamètre externe de la tige d'imagerie (36) ;
un cadre formant barrière (20) destiné à être disposé par-dessus le boîtier d'optique (34), ayant une extrémité distale couplée à l'extrémité proximale du tube allongé, le cadre formant barrière comprenant une cavité formée à l'intérieur de celui-ci dimensionnée pour recevoir le boîtier d'optique du système d'imagerie endoscopique ;
une extrémité proximale de l'élément d'éclairage se terminant en un orifice qui est apte à être en butée contre la source d'éclairage et positionné à proximité de la structure de transmission de lumière tubulaire pleine.

2. Gaine d'éclairage jetable selon la revendication 1, dans laquelle le cadre formant barrière comprend une paroi latérale contenant une fibre de transmission de lumière cylindrique qui s'étend de manière proximale à partir de la structure de transmission de lumière tubulaire pleine.

3. Gaine d'éclairage jetable selon la revendication 1, dans laquelle le tube de transmission de lumière comprend une pluralité de fibres de transmission de lumière (108) disposées à l'intérieur de celui-ci.

4. Gaine d'éclairage jetable selon la revendication 1, dans laquelle de la lumière transmise à travers le tube de transmission de lumière est émise à travers l'extrémité distale (18, 26) sans se propager à travers la fenêtre optiquement transparente.

5. Gaine d'éclairage jetable selon la revendication 1, dans laquelle le tube allongé (12 ; 12') est opaque.

6. Gaine d'éclairage jetable selon la revendication 1, dans laquelle (a) le cadre formant barrière comprend une lumière, formée à travers au moins une partie de celui-ci, pour recevoir la tige d'imagerie (36) ; facultativement, la lumière étant alignée axialement avec le tube allongé (12 ; 12() ; ou (b) le cadre formant barrière comprend une surface orientée de manière proximale ayant un mécanisme de couplage disposé sur celle-ci pour s'accoupler au manche du système d'imagerie endoscopique ; facultativement, (i) le mécanisme de couplage comprenant une monture à baïonnette (58) ou (ii) l'élément d'éclairage (106) s'étendant à travers le cadre formant barrière et comprenant une extrémité de liaison proximale qui se termine sur la surface orientée de manière proximale du cadre formant barrière ; facultativement, le mécanisme de couplage étant configuré pour aligner l'extrémité de liaison proximale de l'élément d'éclairage (106) avec la source d'éclairage dans le manche lorsque le cadre formant barrière est accouplé au manche (32) avec un intervalle entre l'extrémité de liaison proximale et la source d'éclairage (112) de moins de 1 mm dans la direction axiale.

7. Gaine d'éclairage jetable selon la revendication 1, dans laquelle le diamètre externe du tube allongé (12 ; 12') est de moins de 2 mm et a un diamètre interne de 1,5 mm.

8. Gaine d'éclairage jetable selon la revendication 1,
dans laquelle l'élément d'éclairage (106) comprend un connecteur de lumière proximal (200) qui est apte à être en butée contre une source d'éclairage à travers le manche de façon à transmettre de la lumière à l'extrémité distale du tube allongé (12 ; 12') pour éclairer une cible d'imagerie ;
dans laquelle le tube allongé (12 ; 12') est dimensionné de telle sorte que son diamètre externe est de moins d'environ 2 mm ; et
comprenant en outre le cadre formant barrière accouplé à une partie proximale du tube allongé, le cadre formant barrière ayant un élément de liaison proximal (58) pour relier la gaine de manière amovible au manche et aligner le connecteur de lumière avec une source d'éclairage (112) dans le manche (32), un intervalle entre le connecteur de lumière proximal et la source d'éclairage (112) étant de moins de 1 mm dans la direction axiale.

9. Gaine d'éclairage jetable selon la revendication 8, dans laquelle l'élément d'éclairage (106) comprend une pluralité de fibres optiques en suspension à l'intérieur d'un substrat pour former un tube de transmission de lumière plein ; facultativement, (a) la pluralité de fibres optiques et le substrat étant formés à partir d'une matière plastique ; ou (b) le tube de transmission de lumière plein ayant un diamètre externe de moins de 2 mm.

10. Gaine d'éclairage jetable selon la revendication 8, dans laquelle (a) le tube allongé (12 ; 12') est opaque ; ou (b) le tube allongé (12 ; 12') est formé à partir d'un métal.

11. Gaine d'éclairage jetable selon la revendication 8, dans laquelle le tube allongé (12 ; 12') a un diamètre interne de 1,5 mm.

12. Gaine d'éclairage jetable selon la revendication 8, dans laquelle le connecteur de lumière proximal de l'élément d'éclairage est formé sur une surface orientée de manière proximale du cadre formant barrière.

13. Gaine d'éclairage jetable selon la revendication 8, dans laquelle l'élément de liaison proximal (58) est un connecteur à baïonnette.

14. Gaine d'éclairage jetable selon la revendication 1, comprenant en outre un élément structurel cylindrique (100) disposé en relation annulaire avec la structure de transmission de lumière tubulaire pleine (106) ;
ce par quoi, lorsque de la lumière est fournie au connecteur de lumière proximal, la lumière est transmise à travers la structure de transmission de lumière tubulaire pleine (106) et hors de son extrémité distale ; la fenêtre optiquement transparente (16') étant scellée à proximité de l'extrémité distale (18 ; 26) de la surface interne du tube allongé (12 ; 12') ; et
la lumière étant transmise à travers l'extrémité distale (18 ; 26) du tube allongé (12 ; 12') sans passer à travers la fenêtre optiquement transparente (16').

15. Gaine d'éclairage jetable selon la revendication 14, dans laquelle (a) le tube allongé a un diamètre externe de moins de 2 mm ; ou (b) le tube allongé a un diamètre interne de 1,5 mm ; ou (c) l'élément structurel cylindrique est opaque ; ou (d) le cadre formant barrière (20) couplé à l'extrémité proximale du tube allongé ayant un élément de connecteur pour s'accoupler à un boîtier de caméra endoscopique ; facultativement, (a) l'élément d'éclairage cylindrique s'étendant à travers le cadre formant barrière (20) couplé à une paroi latérale de celui-ci ; ou (b) le connecteur de lumière proximal de l'élément d'éclairage cylindrique étant disposé sur une surface orientée de manière proximale du cadre formant barrière.

16. Gaine d'éclairage jetable selon la revendication 1, dans laquelle le tube allongé (12 ; 12') comprend :
un élément tubulaire interne opaque (100) ayant une paroi latérale avec une surface interne, une extrémité proximale, une extrémité distale, une longueur, un diamètre interne et un diamètre externe, le tube allongé étant dimensionné de telle sorte que le diamètre interne est de 1,5 mm, le diamètre interne étant supérieur à un diamètre externe de la tige d'imagerie (36) ; et
la structure de transmission de lumière tubulaire pleine (106) étant disposée de manière concentrique autour de l'élément tubulaire interne et formée à partir d'une pluralité de fibres optiques (108) en suspension dans un substrat (110) dans une forme tubulaire allongée, la structure de transmission de lumière tubulaire pleine (106) étant dimensionnée pour avoir un diamètre externe qui est de moins de 2 mm, la structure de transmission de lumière tubulaire pleine (106) ayant un élément de liaison proximal (200) pour être reliée à une source de lumière (112) dans un boîtier de caméra endoscopique (32) et une extrémité distale pour éclairer une cible d'imagerie ; et le cadre formant barrière (20) comprenant :
a. un intérieur creux pour recevoir le boîtier d'optique endoscopique (34),
b. une extrémité distale couplée à la structure de transmission de lumière tubulaire pleine (106),
c. une paroi latérale contenant la fibre de transmission de lumière cylindrique, et
d. un raccordement d'accouplement (58) formé sur une extrémité proximale et configuré pour s'accoupler à un raccordement correspondant (60) sur le boîtier de caméra endoscopique (32) et aligner l'élément de liaison proximal de la structure de transmission de lumière tubulaire pleine (106) avec une source de lumière sur le boîtier de caméra endoscopique (32) avec un intervalle de moins de 1 mm dans la direction axiale ;
de la lumière transmise à travers la structure de transmission de lumière tubulaire pleine (106) étant transmise à travers son extrémité distale sans se propager à travers la fenêtre optiquement transparente ; facultativement, (a) le cadre formant barrière (20) ayant une forme conique ; ou (b) le raccordement d'accouplement étant un raccordement d'accouplement à baïonnette.
